Europäisches Patentamt

European Patent Office

Office européen des brevets

(11) Publication number: **0 421 235 A2**

## (12) EUROPEAN PATENT APPLICATION

(21) Application number: 90118364.0

(22) Date of filing: 25.09.90

(51) Int. Cl.5: **G01N 33/548**, G01N 33/545

(30) Priority: 27.09.89 US 413569

(43) Date of publication of application:
**10.04.91 Bulletin 91/15**

(84) Designated Contracting States:
**AT BE CH DE DK ES FR GB GR IT LI NL SE**

(71) Applicant: **ABBOTT LABORATORIES**
**CHAD-0377, AP6D/2, One Abbott Park Road**
**Abbott Park, Illinois 60064-3500(US)**

(72) Inventor: **Stimpson, Donald Irvine**
**573 Pine Grove**
**Gurnee, Illinois 60031(US)**
Inventor: **Zakula, Dorothy**
**285 Bonnie Brae**
**Grayslake, Illinois 60030(US)**

(74) Representative: **Modiano, Guido et al**
**MODIANO, JOSIF, PISANTY & STAUB**
**Modiano & Associati Via Meravigli, 16**
**I-20123 Milano(IT)**

(54) Hydrophilic laminated porous membranes and methods of preparing same.

(57) Laminated porous membranes useful in chromatographic assay devices include nitrocellulose or polyvinylidene difluoride (PVDF) membranes laminated to supports. Despite lamination, the membranes remain wettable and the activity of biologically reactive reagents is not impaired, even when organic solvent based adhesives are used, due to the incorporation of an agent of the formula:
$R_1 N(CH_3)_3^+ \cdot SO_4^- R_2$
in a concentration range of from about 0.1% to about 0.2% (w/w) for nitrocellulose, and from about 2% to about 10% (w/w) for PVDF membranes. $R_1$ and $R_2$ are nonpolar, preferably alkyl moieties.

FIG. 1

EP 0 421 235 A2

# HYDROPHILIC LAMINATED POROUS MEMBRANES AND METHODS OF PREPARING SAME

This invention relates to porous membranes useful in immunochromatographic assay devices and, in particular, to laminated nitrocellulose membranes in which hydrophobicity is avoided.

The application is related to two co-owned and concurrently filed applications bearing the same title. The co-pending applications are U.S Serial No. 413,519 and U.S Serial No. 413,571.

## Background

Porous membranes, particularly nitrocellulose membranes, have been used in biochemical procedures such as purification, analytical methodology, and in immunodiagnostics. The well known Western blot is but one example. Nitrocellulose membranes have also been used in immunochromatographic assays such as that disclosed in EP-A-299,428 (Abbott Laboratories).

One problem associated with nitrocellulose membranes is their weak mechanical strength. Another problem associated with chromatographic membranes is evaporation of fluids during the course of chromatography. In order to improve mechanical strength and minimize evaporation, nitrocellulose membranes may be laminated to a support material such as Mylar™. However, the adhesives used in such laminations often affect the hydrophilic properties of the nitrocellulose and render them unstable over time. It has been found that several adhesives used in the lamination of nitrocellulose membranes cause decreased hydrophilicity, as measured by decreasing capillary flow rates through the pores of the membranes.

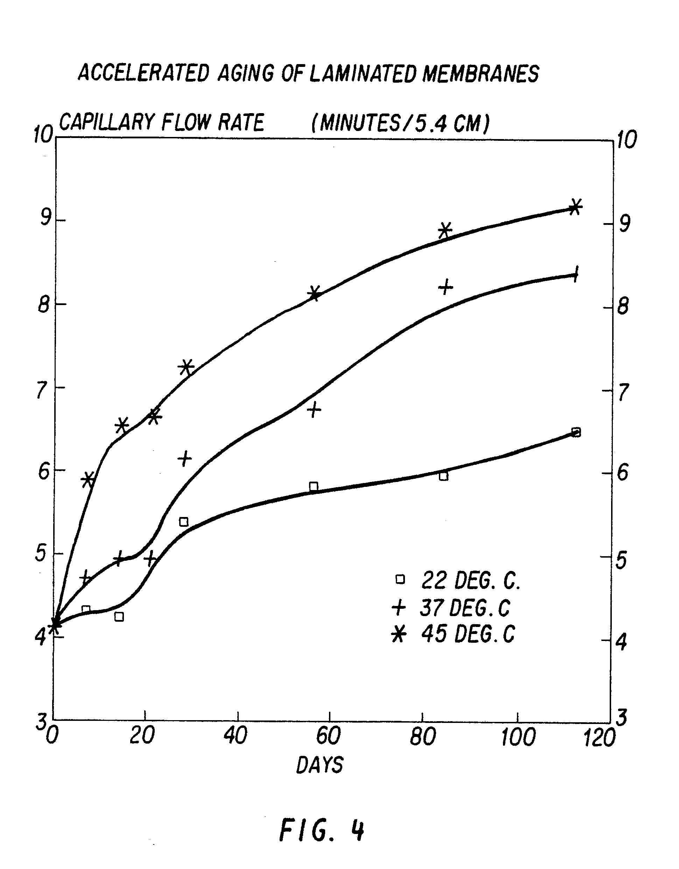

While it is not known for certain why the lamination of porous membranes to a support causes a loss of the hydrophilicity of the membrane, it is speculated that hydrophilicity is lost due to the diffusion or migration of components from the adhesive into the porous membrane. Whatever the mechanism, the loss of hydrophilicity with time is real and is further described in Figure 4 and example 1. This is a critical problem for manufacturing a diagnostic assay since stability must be maintained over a reasonable shelf storage time.

It is known that certain surfactants can be added to the membrane to improve wettability but, at concentrations which render the membranes wettable, the surfactants also disrupt the biologically active reagents present on the membranes. For example, the ability of the membranes to bind protein (eg. antibody) is important for diagnostic applications. It is therefore an important aspect of the invention to provide membranes incorporating surfactants which preserve the hydrophilic nature of the membranes as well as the ability to bind proteins.

It is also an object of the present invention to devise lamination methods and materials which lend mechanical support to nitrocellulose membranes while maintaining their hydrophilic properties. Another purpose of the invention is to employ additional surfactant to enhance the hydrophilicity of the nitrocellulose membranes and render it stable with a wider variety of laminae adhesives.

## Summary of the Invention

In one aspect, the invention relates to a solid phase apparatus useful in a heterogeneous binding assay comprising a porous membrane with which a biologically active reagent is contacted, said porous membrane being laminated on at least one side to a support and incorporating an agent of the formula

$$R_1 N(CH_3)_3^+ \cdot SO_4^- R_2$$

in a concentration of from about 0.1% to about 10% (w/w), wherein $R_1$ represents a straight or branched side chain having from 8 to about 18 carbons and wherein $R_2$ represents a straight or branched alkyl side chain having from 1 to about 5 carbons.

Preferably, the porous membrane comprises nitrocellulose and the final surfactant concentration is from about 0.1% to about 0.2% (w/w). The apparatus alternativley comprises polyvinylidene difluoride membrane and the preferred final surfactant concentration is from about 2% to about 10% (w/w). It is also presently preferred that $R_2$ comprise methyl sulfate, and that $R_1$ comprises $C_{11}H_{23}CONH(CH_2)_3^-$ .

In another aspect, the invention relates to a process for preparing a laminated, wettable solid phase support useful in a diagnostic assay for determining the presence or amount of an analyte, said process comprising:

    a. laminating to a support on at least one side a porous membrane having incorporated therein an agent according to Claim 1 such that the concentration of said agent is from about 0.1% to about 10% (w/w); and

b. contacting a localized site on the porous membrane with a biologically active reagent which retains its activity.

The support may be a semi-rigid polyester or polyolefin plastic and the membrane may be laminated on both sides after a reagent is added to one side. Preferably, the agent is incorporated into the membrane from a water vehicle with a water solvent based adhesive.

In a final aspect, the invention relates to a method for determing the presence or amount of a specific binding ligand in a sample using a porous membrane solid phase, comprising:

a. immobilizing to a localized site on a porous membrane a ligand receptor capable of binding the ligand, wherein the membrane is laminated on at least one side to a support and incorporates an agent according to Claim 1 in a concentration of from about 0.1% to about 10% (w/w);

b. contacting the localized site of the membrane of step a with sample to permit formation of ligand-ligand receptor complex on the membrane; and

c. detecting the presence or amount of complex as a measure of the analyte.

The method of contacting can involve immersing the membrane into sample, or contacting one end of the membrane with sample and allowing sample to wick through the membrane to contact the localized site. In the latter case, the membrane may be laminated on both sides.

The detecting step comprises contacting the complex with a tracer capable of generating a detectable signal. The tracer may be a conjugate of anti-ligand and an enzyme for converting a substrate to a detectable signal; or a conjugate of anti-ligand and a directly detectable colloidal label. The detectable signal is selected from the group consisting of visual color, chemiluminescence, and fluorescence.

Brief Description of Drawings

Figure 1 is a graphic representation of a porous membrane according to the invention which is laminated on one side.

Figure 2 is a graphic representation of a porous membrane according to the invention which is laminated on both sides.

Figure 3 is a graphic representation of the layers of the laminae before it is applied to a porous membrane.

Figure 4 is a graph showing the loss of hydrophilicity upon aging of laminated membranes.

Detailed Description

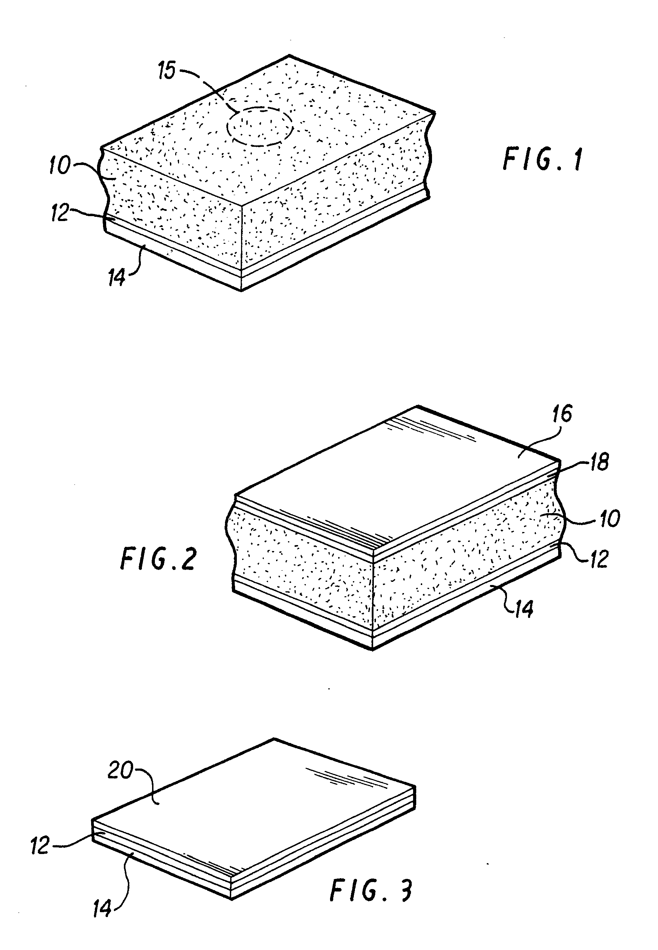

Figure 1 depicts an exemplary embodiment of the invention. An improved porous membrane (10) is laminated on at least one side to a support (14). The membrane (10) is held to the support (14) by an adhesive layer (12). Porous membranes according to the invention have surfactants incorporated into them, which render the membranes wettable yet do not destroy the activity of biologically active reagents in contact therewith.

"Biologically active reagents" include enzymes, nucleic acids and other proteins having activity in their native form. Since the reagent of the preferred embodiment is typically a protein, the term "protein" is often used herein in place of biologically active reagent. However, the invention is not limited to proteins. Similarly, the protein may be immobilized to the membrane or it may merely be in contact with it. It is important that the reagent retain is natural activity while in contact with the membrane and surfactants present there.

Porous membranes of the present invention are useful in a number of biochemical procedures in which proteins must be in contact with or immobilized to a solid phase which is then contacted with a fluid sample. In one system, (see Figure 1) the membrane is laminated on only one side, the protein is applied to a localized site (15) on the surface of the opposite side and fluid is contacted from the opposite side. "Dot blots" (See EP-A 063 810 ) are exemplary of this type of procedure.

In another system, (see Figure 2) the membrane is ultimately laminated on both sides and fluids flow lengthwise through the membrane as in thin layer chromatography. A membrane (10) is laminated on one side, protein is immobilized on a localized site (not shown) on the membrane and a second laminate, consisting of a second support (16) and an adhesive layer (18), is applied to the opposite side. An example of this type of procedure is described in EP 299,428, which is incorporated herein by reference.

The terms "hydrophilic" and "wettable" are frequently used herein and are used interchangeably as antonyms for "hydrophobic". A number of procedures can be used to measure hydrophilicity. Since the membranes of the preferred embodiment resemble thin layer chromatograhic strips, hydrophilicity is here measured as the rate at which the solvent front traverses the membrane strips. Darcy's law provides a rate by relating the distance the front travels to the time t. For a fixed distance, L, the relevant measurement is the time taken for the front to reach L. A relative measure of hydrophilicity is given by comparing the time or "wicking" rate of treated laminated membranes against the time or wicking rate of untreated laminated membranes. Although relative hydrophilicity is sufficient, for purposes of this invention a membrane is considered "hydrophilic" if the flow rate is such that the front traverses a length compatible with visualizing a result (ie., 2 - 10 cm) in a time consistent with rapid diagnostic assays, (ie., less than 10 minutes and preferably less than 5 minutes).

In addition, the ability of the membrane to bind protein is important to this invention. Untreated membranes bind protein, presumably via hydrophobic residues of the protein and possibly through ionic or hydrogen interactions between the protein and the membranes. (Nitrocellulose is known to have a partial negative charge due to the nitrate groups.) The relative ability of a membrane to bind protein can be determined by a number of immunological methods employing antibody as the protein, and determining the relative strength of the signal from a known, constant amount of analyte.

Protein can be contacted with membranes by a number of methods including but not limited to drying, crosslinking, covalent attachment and adsorption. It can be applied from pipette or, more preferably, it can be jetted onto/into a localized site of the membrane prior to lamination. It may be immobilized or move with the solvent front, so long as its activity is retained.

## 1. Membrane Materials:

By "porous membrane" is meant a variety of membranous materials having pores through which fluids can flow by capillary action. Exemplary membranes include nitrocellulose, sintered plastics such as sintered polyethylene or polypropylene, and polyvinylidene difluoride (PVDF). The porous membranes are available in variety of pore sizes ranging from about 0.4 micrometer ("um" or "micron") to about 10 um. Larger pore sizes (i.e. 5 um) are presently preferred for immunodiagnostics because they provide higher fluid flow rates, which lead to more rapidly performed assays.

For the present invention, a preferred porous membrane is nitrocellulose. Nitrocellulose membranes are commercially available from a number of sources, including Gelman Sciences, Ann Arbor, MI; Millipore, Bedford, MA; Schleicher and Schuell (S & S), Keene, NH; Sartorius GmbH, Gottingen, W. Germany; and Micron Separations, Inc. (MSI), Westborough, MA. These commercial sources of nitrocellulose produce membranes having pore sizes from 0.45 um to about 5 um. Commercially available nitrocellulose membranes may incorporate proprietary surfactants as discussed below.

PVDF membranes are available from Millipore. They too are available in several pore sizes, ranging from about 0.22 to about 2.0 um, although other pore sizes may become available. PVDF is generally more hydrophobic than nitrocellulose. As a result, it may bind protiens more tightly, but typically is less wettable and provides poor wicking rates. A hydrophilized product, Durapore is available from Millipore in a variety of pore size ranges.

## 2. Support Laminae:

For purposes of this application, the terms "laminate" or "membrane laminate" shall refer to a membrane bonded to a support. The terms "lamina" or "laminae" shall refer to the support layer to which the membrane is bonded, and may include an associated adhesive layer and a protective release liner.

One method of making membrane laminates involves heat sensitive laminae such as MonokoteTM, available from Top Flight, Chicago, IL. with this particular product, the membrane is laid adjacent the support and heat is applied to the surface to bond the two layers together. This method has the advantage that no additional surfactant is required to maintain the hydrophilicity of the membrane. It remains stable over a reasonable storage time. However, it is not presently a preferred method since the application of heat may inactivate protein already bound to the membrane. In addition, the manufacturing process is facilitated if pressure sensitive laminae are employed. Pressure sensitive laminae adhere to membranes upon the application of pressure.

Laminae useful with the present invention include polyesters such as MylarTM, polyolefins and similar plastics having comparable tensile strength. As previously mentioned, the support lamina is used to give the porous membranes additional mechanical strength and to deter evaporation. As shown in Figure 3, typical laminae provide a support layer (14) coated with a layer of adhesive material (12) which, in turn, is covered by a release liner (20). Typically, the release liner is paper, polyester or similar material having a coating of silicone or other similar material which prevents the adhesive from binding securely to the release liner. "Transfer adhesives" are available having an adhesive layer sandwiched between two release liners. These can be used for special applications where a separate support layer is undesirable.

Polyester support laminae having a support thickness of 50 to 200 mils, preferably 100 to 150 mils are presently preferred due to their ready availability. For example, such laminae are available from Flexcon, Spencer, MA, and Adhesive Research, Inc., Glenrock, PA.

To make a support lamina, an adhesive compound is generally coated onto one surface of a release liner and dried in an oven. The dried adhesive is then contacted with the support layer to form a support lamina.

## 3. Adhesives:

Adhesives are described in Shields, J., Adhesive Handbook , 3rd Ed., (rev. 1985) and typically comprise an adhesive compound combined with tackifiers in a solvent. Adhesive compounds include acrylics, such as polymethylmethacrylate, rubbers and silicones among others. Other adhesive polymers and tackifiers are known to those of ordinary skill in the adhesive arts. The solvent may be organic based or water based. For example, the Flexcon adhesive V23 shown in Table I is an organic solvent based (OSB) adhesive, as are Flexcon V95 and V170, and 3M #396. In contrast, Adhesive Research, Inc (AR) adhesive AS73 (e.g. product No. 7279), casein, polyvinyl acetate (PVA) and polyvinylpyrrolidone (PVP) are useful water solvent based (WSBA) adhesives.

Although the exact compositions of commercially available adhesives are frequently not disclosed by laminae manufactures, the invention can be practiced by using the readily available adhesives referred to herein, which can be ordered by number from the designated manufacturers. Nevertheless, the scope of the invention is not limited to any particular adhesive described herein.

Illustrative adhesives are listed in Table I. It is important to note that the OSB Flexcon laminates worked well (ie., gave improved stability over time while retaining signal indicative of protein binding) with some nitrocellulose lots but worked poorly with others. Specifically, Flexcon PM100CM/V23/71PMO ("71PMO"), Lot No. 1NF3310-33A199011 worked well with S & S nitrocellulose Lot Nos. 4403/8260 and 6419/8921, but worked poorly with S & S Lot Nos. 4406/8221 and 4403/8221. Similarly, Flexcon lamina PM150C/V23/poly SC-9 ("poly SC-9") Lot No. 7ZD3546-33A209841 worked with S & S nitrocellulose Lot No. 6419/8921, but not with any of the remaining three lots tested. In contrast, WSB adhesive AR 7279/AS73 generally gave good stability with most brands of nitrocellulose, even without added surfactants.

It is believed that this result is due to the effect of the adhesive solvent base on the nature and amount of proprietary surfactants contained in the nitrocellulose membranes. Although applicants do not wish to limited by any particular theory or mechanism, it is believed that over time the OSB adhesives may release some hydrophobic organic solvents into the membranes causing the decrease in hydrophilicity. Alternatively, hydrophobicity may result from plasticizers migrating from the support layer through the adhesive layer and into the membrane.

Although the release of water from the WSB adhesives would not have this detrimental effect on the membranes, it was believed that WSB adhesives would dissolve upon contact with aqueous samples, thereby causing delamination and destruction of the laminated apparatus. Surprisingly, it was found that WSB adhesives can be employed successfully without degradation of the membrane laminate.

The adhesive contained in the heat sensitive Monokote™ product also was stable with most nitrocellulose brands tested.

TABLE I

| Membrane (Source) | Proprietary Surfactant | Added* Surfactant | Laminate/ Adhesive | Stability |
|---|---|---|---|---|
| NC (MSI) | unknown usual | none | all 3 tested | good** |
| NC (S&S #4403/8260) | unknown usual | none | AR 7279/AS73 Flexcon 71PMO Flexcon poly SC9 | good fair poor |
| NC (S&S #6419/8921) | unknown two times | none | AR 7279/AS73 Flexcon 71PMO Flexcon poly SC9 | good good good |
| NC (S&S #4406/8221) | unknown usual | none | AR 7279/AS73 Flexcon 71PMO Flexcon poly SC9 | good fair poor |
| NC (S&S #4403/8221) | unknown usual | none | AR 7279/AS73 Flexcon 71PMO Flexcon poly SC9 | good fair poor |
| NC (Sartorius) | unknown usual | none | all 3 tested | poor |
| NC (Sartorius) | unknown usual | 0.1% SDS 0.2% Cyastat | all 3 tested all 3 tested | good good |
| NC (Millipore) | unknown usual | 0.1% SDS | all 3 tested | good |
| NC (Gelman) | unknown usual | | AR 7279/AS73 Flexcon poly SC9 | good poor |
| PVDF (Millipore) | probably none | 6.7% Cyastat (from water) | all 3 tested | good |

* Added surfactant is given in percent w/v of treatment solutions. This can be converted to final concentration w/w by multiplying by a factor of 2.5 for nitrocellulose and 0.97 for PVDF. The conversion factors are determined based on the percent void volume of the membrane, its density, and calculations of the quantity of surfactant present in the volume of a 1% solution which can be imbibed by a given quantity of the membrane. Alternatively, the factors can be determined empirically.

** Stability rating based only on retention of hydrophilic properties. Not all "good" samples gave good protein binding signal.

Thus, WSB adhesives generally form laminates that are stable with commercially available "off-the-shelf" nitrocellulose membranes. Nevertheless, in an effort to secure multiple sources of usable nitrocellulose and multiple sources of usable support laminae, it was desirable to find a way to treat the commercially available nitrocellulose so that more brands remain stably wettable upon lamination and so that more brands retailed the ability to bind proteins. Thus began a search for surfactants which could be added to the nitrocellulose to prevent it from becoming hydrophobic upon lamination with OSB adhesives.

4. Surfactants:

Somewhat surprisingly, it was discovered that not all surfactants will produce a wettable nitrocellulose without affecting the ability of proteins to bind. Typically, surfactants added at concentrations which still permitted protein activity exhibited little or no improvement in the wettability of the membranes over time. As seen from Figure 4, typical laminates showed instability, defined as a decrease in hydrophilicity over time. Since the laminates must have a reasonable shelf life, it is imperative that the wettability of the membranes be maintained. Many of the surfactants tested either failed to improve stability or caused reduced ability to bind protein, or both. Either poor stability or poor protein activity signal rendered a laminate unacceptable.

In addition, it was surprisingly found that the vehicle from which the surfactant is applied to the membrane may also affect its ability to improve the stability of the membranes. While not all surfactants are soluble in all vehicles, as a general rule, surfactants which were applied from a water vehicle performed better than surfactants applied from an isopropanol vehicle. A nonexclusive list of surfactants is given in Table II. They are characterized as non-ionic, cationic, anionic, zwitterionic or antistatic agents. Also given in Table II is the vehicle from which the surfactant was applied and the success of the surfactant as measured by its ability to prevent the membrane from becoming hydrophobic over time while retaining the ability of the membrane to bind proteins. The result is scored a " + " only if the resulting membrane gave good protein activity signal and maintained stable wicking rates over time (taken as indicative of retaining hydrophilicity). The data from Table II is also discussed further in the Examples which follow.

## TABLE II

| Surfactant | Type | Source | Vehicle | Result |
|---|---|---|---|---|
| none (control) | | | water | − |
| none (control) | | | isopropanol | − |
| Pluronic F-68 | N | 1 | water | − |
| Pluronic L-101 | N | 1 | isopropanol | − |
| Pluronic L-62LF | N | 1 | water | − |
| Atmer 110 | S | 2 | water | − |
| Atmer 113 | S | 2 | water | − |
| Atmer 113 | S | 2 | isopropanol | − |
| Zonyl FSN | N | 3 | isopropanol | − |
| Zonyl FSJ | A | 3 | isopropanol | − |
| Zonyl FSP | A | 3 | isopropanol | − |
| Zonyl FSO | N | 3 | isopropanol | − |
| butanol | N | 4 | isopropanol | − |
| 1-octanol | N | 4 | isopropanol | − |
| n-decyl alcohol | N | 4 | isopropanol | − |

| | Type | Source | Vehicle | |
|---|---|---|---|---|
| myristyl alcohol | N | 4 | isopropanol | - |
| stearyl alcohol | N | 4 | isopropanol | - |
| cetyl alcohol | N | 4 | isopropanol | - |
| glycerol | N | | water | - |
| hexadecyltrimethylammonium Br | C | 4 | isopropanol | - |
| dodecyltrimethylammonium Br | C | 4 | isopropanol | - |
| cethydimethylethylammonium Br | C | 4 | isopropanol | - |
| Mackanate DC-30 @1% | | 5 | water | - |
| Mackanate DC-30 @0.1% | | 5 | isopropanol | - |
| Surfonyl PA 104 | N | 6 | isopropanol | - |
| CHAPS | Z | 7 | water | - |
| dioctyl sulfosuccinate | A | 4 | isopropanol | - |
| Aerosol-OT | A | 4 | isopropanol | - |
| Tween-20 | N | 4 | water | - |
| Tween-80 | N | 4 | water | - |
| Triton X405 | N | 9 | water | - |
| Triton X100 | N | 4 | water | - |
| Brij-35 | N | 4 | water | - |
| casein | | 4 | water | - |
| bovine serum albumin | | 4 | water | - |
| pentane sulfonic acid | A | 4 | water | + |
| heptane sulfonic acid | A | 4 | water | + |
| octane sulfonic acid | A | 4 | water | + |
| decane sulfonic acid | A | 4 | water | + |
| dodecane sulfonic acid | A | 4 | water | + |
| sodium dodecyl sulfate | A | 7 | water | + |
| octyl sulfate | A | 4 | isopropanol | - |
| dodecanoic acid | A | 4 | isopropanol | - |
| Cyastat® LS | S | 8 | water | + |
| Cyastat® LS | S | 8 | isopropanol | - |

Type Legend:   N=nonionic, A=anionic, C=cationic, Z=zwitterionic, and S=antistatic.

Source Legend:   1=BASF Performance Chemicals, Parsippany, NJ; 2=ICI Americas, Inc., Wilmington, DE; 3=DuPont, Wilmington, DE; 4=Sigma Chemicals, St. Louis, MO; 5=McIntrye Group, Ltd, Chicago, IL; 6=Air Products, Allentown, PA; 7=Bio Rad, Richmond, CA; 8=American Cyanamid, Polymer Products Division, Wayne, NJ; 9=Aldrich Chemical Company, Milwaukee, WI.

As can be seen from Table II, two classes of agents seem to be successful in improving the stability of the membranes. The first class is the anionic surfactants applied from a water vehicle. The anionics consist of a negatively charged polar head bonded to a non-polar tail. The polar heads typically comprise a sulfate, sulfonic acid, phosphate, or carboxylate group. The non-polar tails comprise hydrocarbon chains having anywhere from 1 to about 16 carbons. The tails may be branched or straight and may also contain other non-polar substituents. Preferably the tail length is 1 to about 12 carbons; most preferably, from 1 to about 8 carbons. Anionic surfactants are commercially available from numerous sources, typically as the sodium or potassium salt. Preferred anionics include alkyl sulfates and alkyl sulfonic acids having from 1-8 carbon atoms.

In addition to the anionic surfactants, one antistatic agent, Cyastat® LS, performed well on both nitrocellulose and PVDF membranes. This class of antistatic agents is referred to herein as "Cyastat-like" and consists of a non-polar chain $R_1$ attached to a trimethylammonium cationic head, coupled with a polar anion bonded to a lower alkyl group $R_2$. $R_1$ comprises a straight or branched side chain having from 8 to about 20 carbons. $R_1$ may also include other substituents, such as the amido moiety of Cyastat LS. $R_2$

8

represents a straight or branched alkyl side chain having from 1 to about 5 carbons. The polar anion may be any of the anions possible with anionic surfactants (see above), although sulfate is presently preferred.

It is not completely understood why these Cyastat-like agents, which appear to be cationic surfactants coupled with an anionic alkyl-sulfate salt, performed well while the bromide salts of similar cationic surfactants failed. However, it is speculated that the anionic surfactant nature of the alkyl-sulfate salt plays an important role. This suggests that anionic surfactants having relatively short non-polar tails may also work very well. It is also possible that the failure of the bromide salts of cationic surfactants was due to the isopropanol vehicle.

The concentration of surfactant used varies from 0.01% to about 10% (w/w) depending on the particular surfactant. In general, for nitrocellulose, anionic surfactants are preferably used in concentrations of 0.1% to about 8% (w/w); most preferably from about 0.25% to about 3.5% (w/w). Since PVDF is more hydrophobic to begin with, slightly higher treatment concentrations (w/v) are preferred but are partially offset by the reduced conversion factor. The final preferred concentrations range from about 1.0% to about 10% (w/w); most preferrably from about 2% to about 5% (w/w).

The Cyastat-like agents are preferably used in a concentration range of from about 0.01% to about 10% (w/w), depending on the membrane. For nitrocellulose membranes, the preferred concentration of these agents is from about 0.1% to about 2.0% (w/w); most preferably from about 0.2% to about 0.5% (w/w). when used with PVDF membranes, the preferred concentrations range from about 2% to about 10% (w/w); most preferably from about 5% to about 9% (w/w). The final concentration (w/w) can be obtained from the treatment solution concentration (w/v) by the constant conversion factor as taught in the note following Table I.

The final membranes tested included whatever proprietary surfactants the particular membrane manufacturer includes, less as much of that surfactant as may have been lost by treatment with additional surfactants in the hands of the inventors. Therefore, the claimed surfactant concentrations given as (%w/w) for anionics include from about 0.01 - to about 3% allowances for anionic surfactants that may have been added to the membranes by the manufacturer. These were estimated based on extraction studies performed on the 5 um commercial membranes, and ranged from about 0.01% to about 11% (w/w) as follows:

| MSI | 9.3% to 11.3% |
| S & S | 0.75% to 2.2% |
| Sartorius | 0.01% to 1.15 |

Since is is doubtful that the Cyastat type agent is incorporated by any membrane manufacturer, no similar allowance is made for the recited percentages of this agent.

5. Methods:

Methods for making membranes according to the invention are evident from the preceding discussion and relevant examples. Generally, an entire sheet of surfactant-treated nitrocellulose is laminated at once and then is cut into strips of desired width. A sheet is placed on a flat surface and the release liner is removed from the desired lamina. The lamina is pressed onto the membrane taking care to avoid wrinkles. A roller capable of applying about 7.0 pounds pressure is used to adhere the lamina to the membrane. Strips of desired width are then cut from the sheet.

Surfactant can be incorporated into the membranes after lamination (of one side) but it is preferable to incorporate surfactant prior to lamination.

Surprisingly, the opposite side of a membrane can also be laminated according to a similar procedure. In this case, any protein to be applied must be done before the second lamination. Since the protein is applied to the surface of the membrane, protein stability problems associated with lamination would be expected to be greatest in the second lamination operation of the same membrane. However, by using the techniques and compositions of the present invention, it has been found that both sides of an immunochromatographic strip can be laminated. This has the added advantage of keeping contaminants out and inhibiting evaporation of sample fluids. When both sides are laminated, a small section (about 1/4 inch) at one end is typically left unlaminated for contact with adjacent members or zones.

Methods of using devices according to the invention are discussed above as well. Additional information

9

is available to one of ordinary skill by resorting to EP-A-299 428, which is incorporated herein in its entirety. The devices are best used in chromatographic immunoassays wherein an antigenic analyte is captured by protein antibody on the membrane. The captured ligand-analyte is then detected by a tracer conjugate of anti-ligand and a signal producer. Signal can be produced directly, as by isotopic labels or colloidal labels, or indirectly as by enzyme labels. All of these techniques are well known in the art, as are competitive assay protocols.

The invention will now be further described by the following examples. The examples are illustrative only and should not be taken to limit the invention in any way.

## EXAMPLES

Example 1.

Nitrocellulose membrane, 5 micron pore size from Schleicher & Schuell, was laminated on both sides using a solvent based acrylic adhesive tape from Flexcon (PM100CM/V23/71PMO) and stored at 22, 37 and 45° C. At various time intervals (0, 7, 14, 21, 28, 56, 84, 112, etc days) the hydrophilicity of the membrane was tested by immersing a strip of laminated membrane 1-3 mm into a test solution (0.1M Tris pH 7.4, 0.9% NaCl, phenol red) and measuring the time required for the solution front to migrate a distance of 5.4 cm. A more hydrophilic membrane requires a shorter time for the liquid to move 5.4 cm. Results (see Fig. 4) indicated that all the laminated membranes became less hydrophilic with time and that increasing the temperature of storage increased the rate at which loss of hydrophilicity occurred.

Example 2.

Nitrocellulose membrane (as in example 1) was laminated on both sides with solvent based acrylic adhesive (V23) tapes from Flexcon, PM100CM/V23/71PMO and PM150C/V23/Poly SC9, and a water based acrylic adhesive (AS73) tape from Adhesive Research, AR7279/AS73. A major difference between the two Flexcon tapes is that the release liner 71PMO is a paper release liner and Poly SC9 is a polyester release liner. AR 7279/AS73 has a polyester release liner. The membranes were incubated at 37° C and tested as described in example 1.

| | Wicking Time (min) to traverse 5.4 cm strip After specified No. of Days Storage at 37° C: | | | | | | | | | |
|---|---|---|---|---|---|---|---|---|---|---|
| Adhesive | 0 | 7 | 14 | 21 | 28 | 35 | 56 | 84 | 112 | 140 |
| Flexcon 71PMO | 4.8 | 7.7 | 6.6 | 6.8 | 6.6 | n/a | 7.2 | 8.1 | 9.1 | 9.3 |
| Flexcon Poly SC9 | 5.9 | 9.3 | 12.6 | 12.6 | 9.7 | 16.2 | | | | |
| AR 7279/AS73 | 6.1 | 5.3 | 5.0 | 6.2 | 5.9 | n/a | 5.6 | 5.7 | 6.0 | 6.3 |

Membrane laminated with AR 7279/AS73 did not become less hydrophilic after 168 days; membrane laminated with PM100CM/V23/71PMO became less hydrophilic by a factor of about two after 140 days; membrane laminated with PM150C/V23/Poly SC9 suffered the greatest loss of hydrophilic character which was a factor of 2.7 after only 35 days. The data indicates that solvent based adhesives can cause the laminated membrane to become hydrophobic. The release liner may play a role in this system by affecting the amount of solvent remaining in the adhesive layer at time of use. One would expect more solvent to be retained in the adhesive with the impermeable polyester release liner Poly SC9 than in the permeable paper liner 71PMO. Also, membrane can be laminated using water based adhesives without loss of the desired hydrophilic character.

Example 3.

Nitrocellulose membrane was laminated and tested as described in example 1 using Flexcon PM150C/V23/Poly SC9 solvent based acrylic adhesive tape. The results indicated that lamination of membrane with this material followed by incubation at 45° C. caused the greatest loss of hydrophilic character.

| | Wicking Time (min) to traverse 5.4 cm strip After specified No. of Days Storage at 45° C: | | | | | | | | | |
|---|---|---|---|---|---|---|---|---|---|---|
| Adhesive | 0 | 7 | 14 | 21 | 28 | 56 | 84 | 112 | 140 | 168 |
| Flexcon 71PMO | 4.1 | 5.9 | 6.5 | 6.6 | 7.2 | 8.2 | 8.9 | 9.2 | 11.5 | 11.9 |
| Flexcon Poly SC9 | 3.6 | 10.0 | 10.4 | 12.6 | 12.3 | | | | | |

Example 4.

Nitrocellulose membrane was impregnated with a single surfactant (see below) by dipping the membrane in a solution of the surfactant such that the membrane was completely wetted by the solution. The membrane was removed from the solution after 5-10 seconds, suspended by a paper clip and allowed to dry at room temperature conditions for 2-20 hours. The resulting membrane was tested as follows. A solution of anti-HCG antibody at 1.2 mg/ml was applied to the membrane in a narrow zone by pumping the solution through a fine capillary tube (Micro ML Tubing, Elmhurst, NY) at a flow rate of 0.05 ml/minute and moving the tubing across the membrane surface at a rate of 0.5 inches/second. The antibody immobilized on the membrane in this narrow zone forms the capture site. The membrane was cut into strips and immunochromatography carried out with a selenium conjugate which binds HCG (see eg. EP-A-299 428). The effect of each surfactant on antibody binding to the membrane was evaluated by the relative amount of selenium conjugate bound to the capture site when 50 mIU HCG urine was used for immunochromatography. Reduction of signal in this test was interpreted as a loss of nitrocellulose antibody binding capacity caused by a blocking effect of the surfactant.

Part A.

Nitrocellulose membrane was treated and tested as described above with each of the following surfactants (from water unless specified otherwise) at a concentration of 1% w/v: Triton X100, Triton X405, Pluronic F68, Pluronic L62F, Pluronic L101, Tween 80, Tween 20, Brij 35, Mackanate DC30, CHAPS, and dioctyl sulfosuccinate (from isopropanol). In each case the surfactant treated membrane resulted in a loss of signal development during immunochromatography.

Part B.

Nitrocellulose membrane was treated and tested as described above with each of the following surfactants (from isopropanol) at a concentration of 0.1% w/v: Mackanate DC30, cetyl alcohol, Zonyl FSO, Zonyl FSN, Zonyl FSP, Zonyl FSJ, and Pluronic L101. Membranes treated with these surfactants showed no reduction in signal development during immunochromatography, however, the membrane wicking rate decreased as a result of the treatment (which is taken to mean the membranes became less hydrophilic as discussed above).

Part C.

Nitrocellulose membrane was treated and tested as described in Part B above, however, 0.5% glycerol was added to the surfactant solution to increase the capillary wicking rate. The resulting membranes showed no decrease in signal development during immunochromatography. (But see example 5 as to effect on hydrophilicity.)

Part D.

Nitrocellulose membrane was treated and tested as described above with each of the following surfactants from an isopropanol solution at a concentration of 1% w/v: dodecyltrimethylammonium bromide, cetyltrimethylethylammonium bromide hexadecyltrimethylammonium bromide, and Surfonyl 104PA. The resulting membranes showed no decrease in signal development during immunochromatography. (But see example 5 as to effect on hydrophilicity.)

Part E.

Nitrocellulose membrane was treated and tested as described above with each of the following surfactants (in water solution):
1% pentane sulfonic acid, 1% heptane sulfonic acid, 1%, octane sulfonic acid, 1% decane sulfonic acid, 0.1% dodecane sulfonic acid, 0.1% sodium dodecyl sulfate, and 0.2% Cyastat LS. The resulting membranes showed no decrease in signal development during immunochromatography.

Example 5.

Membranes produced as described in example 4, Parts C and D were tested as described in example 3. As a result of lamination with Flexcon PM150C/V23/Poly SC9, all membranes suffered such a loss of hydrophilic character that after 14 days the flow rate was unacceptably slow or variable. For purposes of these studies, a flow time of more than 10 minutes for a 5.4 cm strip or a flow rate change of more than 20% was considered unacceptable. These studies were stopped after concluding the laminate was unacceptable.

Example 6.

Membranes produced as described in example 4, Part E were tested as described in example 3. All membranes retained their hydrophilic character after lamination with Flexcon PM150C/V23/Poly SC9 and accelerated aging at 45° C.

| | Wicking Time (min) to traverse 5.4 cm strip After specified No. of Days Storage at 45° C: | | | | |
|---|---|---|---|---|---|
| Surfactant | 0 | 7 | 14 | 21 | 28 |
| pentane sulfonic | 4.1 | 5.5 | 5.4 | 5.6 | 5.6 |
| heptane sulfonic | 4.9 | 5.3 | 5.2 | 5.2 | 5.3 |
| octane sulfonic | 5.1 | 5.6 | 5.4 | 5.6 | 5.7 |
| decane sulfonic | 5.7 | 6.4 | 6.0 | 6.3 | 6.2 |
| dodecane sulfonic | 6.2 | 6.4 | 6.0 | 6.3 | 6.3 |
| dodecyl sulfate | 6.3 | 7.1 | 7.0 | 6.4 | 6.0 |
| Cyastat | 5.5. | 6.3 | 6.2 | | |

This is interpreted to mean that these surfactants do not interfere with nitrocellulose antibody binding and confer resistance to the loss of hydrophilic character induced by a solvent based acrylic adhesives.

Example 7.

Nitrocellulose membrane was treated and tested as described in example 4, Part E with 1% Cyastat LS in either isopropanol or water solution, and subsequently tested as described in example 3. The membrane treated from the isopropanol solution lost hydrophilic character upon lamination and aging with PM150C/V23/Poly SC9, whereas the membrane treated from the water solution did not.

| Cyastat from: | Wicking Time to traverse specified distance After specified No. of Days Storage: | | | | | | | |
|---|---|---|---|---|---|---|---|---|
| | 0 | 7 | 14 | 21 | 28 | 56 | 84 | 112 |
| water solution (5.4 cm in min) | 5.1 | 6.2 | 5.7 | 6.6 | 6.3 | 6.2 | 6.2 | 6.1 |
| isopropanol | 0.4 | 5.4 | 4.5 | | | | | |
| (1.4 cm in min) | (unacceptable when extrapolated to 5.4 cm.) | | | | | | | |

Example 8.

Nitrocellulose membrane (S&S 5 micron) was laminated with an organic solvent rubber based adhesive (3M-#396), stored at 37° C and tested for capillary wicking rate.

| Adhesive | Wicking Time (min) to traverse 5.4 cm strip After specified No. of Days Storage: | | | | |
|---|---|---|---|---|---|
| | 0 | 7 | 14 | 21 | 28 |
| 3M #396 | 4.7 | 22.2 | 26.3 | 33.3 | 37.7 |

Example 9.

Nitrocellulose membrane was laminated with an organic solvent acrylic based adhesive (Flexcon V95), stored at 37° C and tested for capillary wicking rate.

| Adhesive | Wicking Time (min) to traverse 5.4 cm strip After specified No. of Days Storage: | | | |
|---|---|---|---|---|
| | 0 | 7 | 14 | 21 |
| Flexcon V-95 | 5.0 | 8.7 | 9.3 | 10.3 |

Example 10.

Nitrocellulose membrane was laminated with an organic solvent acrylic based adhesive (Flexcon V170), stored at 37° C and tested for capillary wicking rate.

| Adhesive | Wicking Time (min) to traverse 5.4 cm strip After specified No. of Days Storage: | | | | | | | | |
|---|---|---|---|---|---|---|---|---|---|
| | 0 | 7 | 14 | 21 | 28 | 56 | 84 | 112 | 140 |
| Flexcon V170 | 4.2 | 6.2 | 7.1 | 9.1 | 7.3 | 8.9 | 10.0 | 9.7 | 11.9 |

Example 11.

Nitrocellulose membrane was laminated with a heat activated adhesive (MonoKote), stored at 37° C and tested for capillary wicking rate.

| | Wicking Time (min) to traverse 5.4 cm strip After specified No. of Days Storage: | | | |
|---|---|---|---|---|
| Adhesive | 0 | 7 | 14 | 21 |
| Monokote | 4.1 | 4.2 | 4.4 | 4.5 |

Example 12.

Nitrocellulose is laminated with a hot-melt adhesive consisting of a layer of polyethylene bonded to polyester. The hot-melt adhesive is a thermoplastic of 100% solids with a melt temperature ranging from 65-90° C. This lamination process should not affect membrane flow rate because their is no opportunity for hydrophobic, organic solvents to migrate from the bonding layer into the membrane.

Example 13.

Nitrocellulose is laminated with a water based casein adhesive consisting of a layer of viscous casein solution bonded to a polyester support. Such a material is produced by applying a thin layer of a 20% solution of casein in water to polyester and evaporating water from the layer to reach a final concentration of 70-90%. Lamination with this adhesive material should not cause a loss of hydrophilic character in the membrane because migration of water from the adhesive to the membrane would increase the degree of membrane hydration.

Example 14.

Nitrocellulose is laminated with a water based polyvinylpyrrolidone (PVP) adhesive consisting of a viscous PVP solution bonded to a polyester support. Such a material is produced by applying a thin layer of 20-30% w/v PVP (molecular weight 3,000-5,000) and evaporating water from the layer to reach a final concentration of 70-90% w/v. Lamination with this material should not cause loss of hydrophilic character for reasons given in example 13.

Example 15.

Nitrocellulose is laminated with an adhesive made from a water emulsion of polyvinyl acetate (PVA) particles. A 70% solids water solution of PVA particles, 1-50 micron diameter, with 0.5% sodium dodecyl sulfate stabilizing surfactant is applied in a thin layer to a polyester support and the water evaporated to a final concentration of 90-99% solids. Lamination with this material should not cause loss of hydrophilic character for reasons given in example 13.

Example 16.

A nitrocellulose web 7.3 inches wide moving at 0.5 feet per minute was drawn through a bath of one of several solutions of Cyastat LS in concentrations as follows: 0.1, 0.2, 0.3, 0.4 and 0.5% (w/v). The immersion path length was about 3-4 inches giving a resident time of 30-40 seconds. The web was then dried in a drying tunnel at 60° C. for approximately 10 min. Strips cut from the sheet were tested as in examples 3 and 4 above using poly SC-9 laminates stcred at 37° C. The signals from untreated control and 0.1% and 0.2% samples were good; the signals from 0.3% and 0.4% treated samples were fair; and the signal from the 0.5% treated sample was poor. The hydrophilicity stability was as follows:

| Cyastat Concentration | Wicking Time (min) to traverse 5.4 cm strip After specified No. of Days Storage at 37° C.: | | | |
|---|---|---|---|---|
| | 0 | 5 | 7 | 14 |
| 0.1% | 6.8 | 12.7 | 12.0 | 12.2 |
| 0.2% | 5.5 | 6.3 | 6.3 | 6.2 |
| 0.3% | 4.3 | 5.3 | 5.3 | 5.2 |
| 0.4% | 4.8 | 4.7 | 4.7 | 4.4 |
| 0.5% | 4.8 | 4.6 | 4.8 | 4.6 |

Example 17.

Polyvinylidene difluoride (PVDF) membrane 2.0 micron, was obtained from Millipore. This material is the hydrophobic precursor to Millipore's hydrophilic Durapore material. The membrane as supplied could not be wet with a water solution, hence, antibody reagent cannot conveniently be applied to the membrane. Hydrophilic Durapore displayed very low protein binding and, hence, is not useful for immobilization of antibody reagent by adsorption.

Example 18.

Hydrophobic PVDF membrane 2.0 micron, was impregnated with a solution of 1% w/v Pluronic L101 and dried. The resulting membrane could be wet by an aqueous solution of anti-HCG antibody, however, no signal was developed during 10 minutes of immunochromatography with anti-HCG selenium conjugate at a 500 mIU analyte concentration. Presumably, the surfactant allows wetting but blocks protein binding.

Example 19.

Hydrophobic PVDF membrane 2.0 micron, was impregnated with a solution of 6.7% w/v Cyastat LS and dried. A 1 uL volume of anti HCG antibody (3.3 mg/ml) was applied to the resulting membrane and immunochromatography carried out using anti-HCG selenium conjugate with 500 mIU HCG urine sample. Results showed signal development equivalent to that observed using nitrocellulose membrane.

Example 20.

PVDF membrane is dipped into isopropanol to obtain complete wetting of the material. The isopropanol is washed out by soaking the wetted membrane in a water bath with several exchanges of the washing water. The membrane is then impregnated with sodium dodecyl sulfate (SDS) surfactant by dipping the membrane in a 5% w/v water solution of SDS for a sufficient time to allow diffusion of the surfactant into the void structure of the membrane. The resulting membrane impregnated with the 5% water-SDS solution is removed from the bath and dried. Presuming the binding of protein to PVDF is similar to that of nitrocellulose, this membrane should easily wet and immobilize aqueous solutions of antibody. This is a general means of introducing surfactants which are soluble in water but not soluble in organic solvents like isopropanol into a hydrophobic PVDF membrane.

**Claims**

1. A solid phase apparatus useful in a heterogeneous binding assay comprising a porous membrane with which a biologically active reagent is contacted, said porous membrane being laminated on at least one side to a support and incorporating an agent of the formula

$R_1 N(CH_3)_3^+ \cdot SO_4^- R_2$

in a concentration of from about 0.1% to about 10% (w/w), wherein $R_1$ represents a straight or branched side chain having from 8 to about 18 carbons and wherein $R_2$ represents a straight or branched alkyl side chain having from 1 to about 5 carbons.

2. The apparatus according to claim 1 wherein the porous membrane comprises nitrocellulose.

3. The apparatus according to claim 2 wherein the surfactant concentration is from about 0.1% to about 2% (w/w).

4. The apparatus according to claim 1 wherein the porous membrane comprises polyvinylidene difluoride.

5. The apparatus according to claim 4 wherein the surfactant concentration is from about 2% to about 10% (w/w).

6. A process for preparing a laminated, wettable solid phase support useful in a diagnostic assay for determining the presence or amount of an analyte, said process comprising:

    a. laminating to a support on at least one side a porous membrane having incorporated therein an agent according to claim 1 such that the concentration of said agent is from about 0.1% to about 10% (w/w); and

    b. contacting a localized site on the porous membrane with a biologically active reagent which retains its activity.

7. The process according to claim 6 further comprising a step of laminating the opposite side of the porous membrane.

8. The process according to claim 6 wherein said laminating of step b is done with an organic solvent based adhesive.

9. A method for determining the presence or amount of a specific binding ligand in a sample using a porous membrane solid phase, comprising:

    a. immobilizing to a localized site on a porous membrane a ligand receptor capable of binding the ligand, wherein the membrane is laminated on at least one side to a support and incorporates an agent according to claim 1 in a concentration of from about 0.1% to about 10% (w/w);

    b. contacting the localized site of the membrane of step a with sample to permit formation of ligand-ligand receptor complex on the membrane; and

    c. detecting the presence or amount of complex as a measure of the analyte.

10. The method according to claim 9 wherein the contacting of step b comprises immersing the membrane into sample.

11. The method according to claim 9 wherein the contacting of step b comprises contacting one end of the membrane with sample and allowing sample to wick through the membrane to contact the localized site.

FIG. 1

FIG.2

FIG. 3

FIG. 4